# EUROPÄISCHE PATENTSCHRIFT

(11) **EP 3 957 347 B1**
(45) Veröffentlichungstag und Bekanntmachung des Hinweises auf die Patenterteilung: **16.10.2024**
(21) Anmeldenummer: 21190924.7
(22) Anmeldetag: 12.08.2021
(51) Int. Cl.: A61M 16/00, A61M 16/20

(54) **BEATMUNGSGERÄT FÜR HIGH-FLOW-SAUERSTOFFTHERAPIE**
VENTILATOR FOR HIGH-FLOW OXYGEN THERAPY
RESPIRATEUR POUR L'OXYGÉNOTHÉRAPIE À DÉBIT ÉLEVÉ

(30) Priorität: 20.08.2020 DE 102020121871
(43) Veröffentlichungstag der Anmeldung: 23.02.2022
(73) Patentinhaber: Drägerwerk AG & Co. KGaA, 23558 Lübeck (DE)
(72) Erfinder: Ralfs, Frank, 23558 Lübeck (DE); Neumann, Andreas, 23558 Lübeck (DE)

(56) Entgegenhaltungen:
- EP-A2- 1 205 203
- WO-A1-2009/094532
- US-A1- 2002 017 300
- US-A1- 2020 129 719

## Beschreibung

Die Erfindung betrifft ein Beatmungsgerät zur Beatmung eines Patienten durch eine High-Flow-Sauerstofftherapie über ein Schlauchsystem. Weiterhin betrifft die Erfindung ein Beatmungssystem zur Beatmung eines Patienten durch eine High-Flow-Sauerstofftherapie.

Bei der High-Flow-Sauerstofftherapie werden typischerweise an einem entsprechenden Beatmungsgerät eine Sauerstoffkonzentration und ein Gasfluss eingestellt. Dieser Gasfluss wird dann über eine Patientenschnittstelle zu einem zu beatmeten Patienten geführt. Der Gasfluss erfolgt dabei über ein Schlauchsystem, welches zumindest einen Schlauch umfasst. Grundsätzlich sind für die High-Flow-Sauerstofftherapie Ein-Schlauch-Systeme und Zwei-Schlauch-Systeme mit dazwischenliegendem Y-Stück bekannt. Die Patientenschnittstelle sorgt dabei für eine Beatmung mittels eines dynamischen Überdrucks, wie er beispielsweise durch sogenannte Prongs bereitgestellt werden kann. Dabei fließt der Gasfluss größtenteils am Patienten vorbei in die Umgebung. Die Patientenschnittstelle dichtet die Atemwege des Patienten nicht ab, wobei der Grad der entsprechenden Leckage zwischen dem zu beatmenden Patienten und der Patientenschnittstelle den bereitgestellten Druck innerhalb der Atemwege des Patienten bestimmt. Gleichzeitig wird durch Turbulenzen des Gases möglicher Totraum gespült.

Für eine derartige Beatmung ist die Verwendung eines federvorgespannten Ventils bekannt, über das sichergestellt wird, dass bei Erreichen einer Druckgrenze im Schlauchsystem das entsprechend überschüssige Gas über das federvorgespannte Ventil abgegeben wird.

Aus EP 1 205 203 A2 ist ein Ausatemstrommessgeber mit einer Klappe bekannt, welcher eingerichtet ist stromaufwärts und stromabwärts der Klappe eine Druckdifferenz zu erzeugen. Der Ausatemstrommessgeber weist Mittel zum Messen der Druckdifferenz und Mittel zum Berechnen einer Durchflussrate auf Grundlage der Druckdifferenz auf.

Aus WO 2009 / 094 532 A1 ist eine Nasenkanüle bekannt.

Aus US 2002 / 0 017 300 A1 ist eine Vorrichtung und ein Verfahren für die Zufuhr von Atemgas zu einer Person bekannt. Dabei ist eine Sauerstoffzufuhrvorrichtung eingerichtet der Person während einer Einatemphase einen höheren Sauerstofffluss zuzuführen.

Aus US 2020 / 0 129 719 A1 ist ein System zum selektiven Verabreichen eines hohen Sauerstoffflusses bekannt, wobei eine Quelle komprimierten Sauerstoffs mit einer ersten Nasenkanüle über ein Ventil zur Steuerung des Sauerstoffflusses verbunden ist. In einer zweiten Nasenkanüle ist ein Drucksensor angeordnet.

Aufgabe der vorliegenden Erfindung ist es, ein verbessertes Beatmungsgerät, insbesondere ein besonders sicheres Beatmungsgerät für eine High-Flow-Sauerstofftherapie, bereitzustellen.

Erfindungsgemäß wird zur Lösung dieser Aufgabe ein Beatmungsgerät zur Beatmung eines Patienten durch eine High-Flow-Sauerstofftherapie über ein Schlauchsystem, vorgeschlagen, wobei das Beatmungsgerät mindestens ein Sensorelement, mindestens ein ansteuerbares Inspirations- oder Exspirationsventil und eine Steuereinheit aufweist.

Das mindestens eine Sensorelement ist angeordnet und ausgebildet, eine Messgröße innerhalb des Schlauchsystems zu bestimmen und auszugeben, wobei die Messgröße einen Gasfluss innerhalb des Schlauchsystems indiziert.

Das mindestens eine ansteuerbare Inspirations- und/oder Exspirationsventil, insbesondere das mindestens eine elektrisch ansteuerbare Inspirations- oder Exspirationsventil, ist angeordnet und ausgebildet, einen Fluss eines Atemgases aus einem Beatmungskreis des Beatmungsgerätes heraus zu ermöglichen.

Die Steuereinheit regelt einen durch das Beatmungsgerät bereitgestellten Beatmungsdruck derart über das mindestens eine Sensorelement und das mindestens eine Inspirations- oder Exspirationsventil, dass ein vorbestimmter Maximaldruck in einem vorbestimmten Bereich des Schlauchsystems nicht überschritten wird.

Im Rahmen der Erfindung wurde erkannt, dass hohe Drücke unmittelbar am Patienten zur Sicherung der Patientengesundheit vermieden werden müssen. Hierfür wird die Verwendung eines ansteuerbaren Inspirations- oder Exspirationsventils vorgeschlagen, über das ein gleichmäßig hoher Gasfluss während der High-Flow-Sauerstofftherapie über die gesamte Dauer der Therapie unter Berücksichtigung des vorbestimmten Maximaldrucks sichergestellt ist.

Durch die Ansteuerbarkeit des Inspirations- oder Exspirationsventils kann automatisiert ein für den zu beatmenden Patienten gefährliches Druckniveau während der Beatmung vermieden werden, ohne dass dafür eine Veränderung des bereitgestellten Gasflusses notwendig ist. So kann die angestrebte High-Flow-Sauerstofftherapie über die komplette angestrebte Dauer der Therapie ausgeführt werden, ohne dass das Risiko eines zu hohen Drucks am Patienten besteht.

Das Sensorelement umfasst zumindest einen Sensorikteil, und einen Verbindungsteil, der den Sensorikteil mit der Steuereinheit direkt oder indirekt verbindet. Vorzugsweise ist das mindestens eine Sensorelement ein Sensor, wie beispielsweise ein Drucksensor oder ein Flusssensor. Der genaue Aufbau solcher Sensoren ist bekannt und wird daher im Folgenden nicht erläutert.

Ein Inspirationsventil ist ein Ventil durch welches ein für die Inspiration des Patienten vorgesehener Gasfluss entweichen kann. Ein Exspirationsventil ist ein Ventil, durch welches ein Ausatemgas des Patienten entweichen kann.

Vorzugsweise ist ein Inspirationsventil im Bereich eines Inspirationsschlauch und ein Exspirationsventil im Bereich eines Exspirationsschlauchs angeordnet.

Vorzugsweise umfasst die Steuereinheit ein Speichermodul, auf dem der vorbestimmte Maximaldruck hinterlegt ist. Beim Regeln des Beatmungsdruckes greift die Steuereinheit auf diesen hinterlegten vorbestimmten Maximaldruck zu, und vergleicht diesen mit einem Wert, der durch die ausgegebene Messgröße indiziert wird. Der vorbestimmte Maximaldruck ist dabei ein Wert, der einen maximal vorliegenden Druck indiziert. Der Maximaldruck ist in einer Ausführungsform eine Größe, aus dem sich unmittelbar der maximal in dem vorbestimmten Bereich des Schlauchsystems vorliegende Druck bestimmen lässt.

Die Bestandteile des erfindungsgemäßen Beatmungsgeräts können in einem gemeinsamen Gehäuse oder zumindest teilweise beabstandet voneinander angeordnet sein. Das Schlauchsystem ist hierbei kein Teil des erfindungsgemäßen Beatmungsgerätes.

Der Beatmungskreis kann im Sinne der Erfindung offen sein, beispielsweise für eine Ein-Schlauch-Struktur des Schlauchsystems, oder geschlossen sein, wie beispielsweise beim Vorsehen eines Inspirations- und eines Exspirationsschlauches.

Nachfolgend werden bevorzugte Ausführungsformen des erfindungsgemäßen Beatmungsgerätes beschrieben.

In einer besonders bevorzugten Ausführungsform ist das Beatmungsgerät ausgebildet, das mindestens eine Inspirations- oder Exspirationsventil geschlossen zu halten, falls der vorbestimmte Maximaldruck nicht in dem vorbestimmten Bereich des Schlauchsystems erreicht ist. In dieser Ausführungsform beeinflusst das Inspirations- oder Exspirationsventil nicht den Gasfluss während der High-Flow-Sauerstofftherapie, solange kein kritischer Druck in dem vorbestimmten Bereich vorliegt.

Vorzugsweise ist das mindestens eine Sensorelement innerhalb eines Exspirationsschlauchs angeordnet. In dieser Ausführungsform wird eine Messgröße bestimmt, die eine Menge von an einer Patientenschnittstelle vorbeiströmendem Gas pro Zeiteinheit indiziert. So kann durch das Sensorelement festgestellt werden, ob sich gerade eine ungewöhnlich große Gasmenge im Bereich des Exspirationsschlauchs sammelt, was beispielsweise ein Beleg für eine zu dicht am Patienten anliegende Patientenschnittstelle sein kann. Ohne Leckage führt der Gasfluss im Rahmen der High-Flow-Sauerstofftherapie typischerweise zur einem zu großen auf den Patienten wirkenden Gasdruck. Daher kann durch das Erkennen einer zu geringen Leckage besonders zuverlässig durch die Steuereinheit entschieden werden, dass das entsprechende Inspirations- oder Exspirationsventil derart angesteuert werden muss, dass der am Patienten anliegende Druck reduziert ist. Erfindungsgemäß wird dabei nicht der Gasfluss am Patienten reduziert.

Erfindungsgemäß führt die Steuereinheit die Regelung des bereitgestellten Beatmungsdruckes aus, ohne hierfür eine Leistung eines Gebläses des Beatmungsgerätes oder dergleichen zu verändern. Hierdurch wird ein konstanter Gasfluss am Patienten sichergestellt, wie er für die High-Flow-Sauerstofftherapie notwendig ist. Die Regelung durch die Steuereinheit erfolgt dabei vorzugsweise ausschließlich durch das Öffnen des entsprechenden Inspirations- oder Exspirationsventils bei Vorliegen eines den vorbestimmten Maximaldruck überschreitenden Druckes innerhalb des vorbestimmten Bereichs des Schlauchsystems.

Vorzugsweise ist das Inspirations- oder Exspirationsventil ein elektrisch ansteuerbares Inspirations- oder Exspirationsventil. Hierdurch kann besonders zuverlässig und schnell die Regelung durch die Steuereinheit erfolgen. In einem alternativen oder ergänzenden Ausführungsbeispiel ist das Inspirations- oder Exspirationsventil pneumatisch oder auf andere Weise mechanisch durch die Steuereinheit ansteuerbar.

Besonders bevorzugt ist das mindestens eine Sensorelement ein Drucksensor und/oder ein Flusssensor. Ein derartiges Sensorelement kann direkt, ohne zeitaufwändige Algorithmen zur Signalauswertung, den vorliegenden Druck und/oder über einen bestimmten Gasfluss den vorliegenden Druck anzeigen.

Vorzugsweise ist das mindestens eine Sensorelement in dem vorbestimmten Bereich des Schlauchsystems angeordnet. Hierdurch kann direkt aus den bestimmten Sensordaten ermittelt werden, ob der vorbestimmte Maximaldruck überschritten ist oder nicht. In einer alternativen oder ergänzenden Ausführungsform ist das mindestens eine Sensorelement beabstandet von dem vorbestimmten Bereich des Schlauchsystems angeordnet. In dieser Ausführungsform muss die bestimmte Messgröße umgerechnet werden zu einem Wert, der den Druck in dem vorbestimmten Bereich des Schlauchsystems indiziert.

In einer besonders bevorzugten Ausführungsform ist die Steuereinheit weiterhin ausgebildet, den bereitgestellten Beatmungsdruck basierend auf einer vorbestimmten Kalibrierungsinformation zu regeln. Die vorbestimmte Kalibrierungsinformation ist vorzugsweise in einem Speichermodul der Steuereinheit hinterlegt. Über die vorbestimmte Kalibrierungsinformation kann besonders zuverlässig von der bestimmten Messgröße auf den Druck innerhalb des vorbestimmten Bereichs des Schlauchsystems geschlossen werden.

In einer besonders vorteilhaften Variante der vorhergehenden Ausführungsform indiziert die Kalibrierungsinformation einen Zusammenhang zwischen dem in dem vorbestimmten Bereich des Schlauchsystems vorliegenden Druck und dem durch den mindestens einen Flusssensor gemessenen Gasfluss. Ein derartiger Zusammenhang kann durch eine funktionelle Zuordnung, durch eine grafische Zuordnung über ein Diagramm und/oder über eine vorbestimmte Zuordnung von Wertepaaren erfolgen. Die Kalibrierungsinformation berücksichtigt dabei vorzugsweise geometrische Eigenschaften des Schlauchsystems. In einer bevorzugten Ausführungsform wird Kalibrierungsinformation im Rahmen eines Testbetriebs des Beatmungsgeräts vor einem darauffolgenden Einsatz des Beatmungsgeräts am zu beatmenden Patienten ermittelt.

In einer besonders vorteilhaften Variante der vorhergehenden Ausführungsform basiert die Kalibrierungsinformation weiterhin auf einem Schlauchwiderstand, insbesondere einem vorbestimmten Schlauchwiderstand, des Schlauchsystems. In dieser Variante wird die Geometrie zumindest eines Teils des Schlauchsystems berücksichtigt bei dem Bestimmen der Kalibrierungsinformation. Hierdurch kann besonders vorteilhaft aus der Messgröße, wie beispielsweise aus einem Gasfluss oder einem Druck auf den in dem vorbestimmten Bereich vorliegenden Druck geschlossen werden. Besonders bevorzugt wird die Messgröße hierbei in dem vorbestimmten Bereich ermittelt. Beispielsweise kann anhand des Schlauchwiderstands über einen gemessenen Gasfluss direkt auf den durch den Gasfluss indizierten Druck innerhalb dieses Schlauchbereichs geschlossen werden.

Besonders bevorzugt ist der vorbestimmte Bereich des Schlauchsystems ein zum Patienten weisendes Ende eines Inspirations- oder Exspirationsschlauchs. In dieser Ausführungsform kann besonders zuverlässig ein zu hoher Druck am Patienten vermieden werden, da der vorbestimmte Maximaldruck erfindungsgemäß direkt im Bereich des Patienten, nämlich an einem entsprechenden Ende des Inspirations- oder Exspirationsschlauchs, nicht überschritten wird. Sollte dieser vorbestimmter Maximaldruck in diesem Bereich am Patienten überschritten werden, wird durch die Steuereinheit das Inspirations- oder Exspirationsventil geöffnet und der Druck zum Schutz des Patienten reduziert.

In einer vorteilhaften Ausführungsform ist das mindestens eine Inspirations- oder Exspirationsventil zusammen mit der Steuereinheit in einem Gehäuse des Beatmungsgerätes angeordnet. In dieser Ausführungsform kann vorteilhaft das erfindungsgemäße Beatmungsgerät für unterschiedliche Schlauchsysteme verwendet werden, ohne dass die Funktionsweise des Inspirations- oder Exspirationsventils dadurch beeinträchtigt ist. Vorzugsweise ist hierbei das Inspirations- oder Exspirationsventil im Bereich einer Gaszuführung zu dem Schlauchsystem oder einer Gasrückführung von dem Schlauchsystem angeordnet.

In einer vorteilhaften Ausführungsform kann das mindestens eine Inspirations- oder Exspirationsventil nur in einer offenen Ventilstellung und in einer geschlossenen Ventilstellung vorliegen in dieser Ausführungsform ist das Inspirations- oder Exspirationsventil besonders einfach ausgestaltet. Hierdurch kann dieses Ventil besonders robust und günstig in der Herstellung sein. Ein derartiges Ventil mit nur zwei verschiedenen Ventilstellung kann über eine besonders hohe Ausfallsicherheit verfügen, was vorteilhaft für die Zuverlässigkeit des erfindungsgemäßen Beatmungsgeräts ist.

Der vorbestimmte Maximaldruck ist vorzugsweise individuell einstellbar. Vorzugsweise liegt der vorbestimmte Maximaldruck zwischen 5mbar und 25mbar, insbesondere zwischen 10 mbar und 20 mbar, besonders bevorzugt bei etwa 12 mbar. Besonders bevorzugt ist der vorbestimmte Maximaldruck zwischen 5mbar und 25mbar, insbesondere zwischen 10 mbar und 20 mbar, individuell einstellbar.

Vorzugsweise ist das Beatmungsgerät weiter ausgebildet, über eine Anzeigeeinheit anzuzeigen, wenn durch die Steuereinheit eine Betätigung des Inspirations- oder Exspirationsventil stattgefunden hat. Hierdurch ist ein Nutzer darüber informiert, dass der vorbestimmte Maximaldruck in dem vorbestimmten Bereich des Schlauchsystems überschritten wurde. Dies kann beispielsweise anzeigen, dass an einer Schnittstelle des Schlauchsystems zum Patienten eine gewünschte Leckage nicht vorhanden ist.

Gemäß einem zweiten Aspekt der Erfindung wird zur Lösung der oben genannten Aufgabe ein Beatmungssystem zur Beatmung eines Patienten durch eine High-Flow-Sauerstofftherapie vorgeschlagen, mit einem erfindungsgemäßen Beatmungsgerät gemäß mindestens einer der vorhergehenden Ausführungsformen, und einem Schlauchsystem mit einem Inspirationsschlauch und einem Exspirationsschlauch, wobei das Schlauchsystem zum Bereitstellen eines Beatmungskreises an dem Beatmungsgerät angeschlossen ist.

Das erfindungsgemäße Beatmungssystem umfasst das erfindungsgemäße Beatmungsgerät und damit auch sämtliche Vorteile dieses Beatmungsgerätes.

Weiterhin kann das Schlauchsystem hinsichtlich seiner Geometrie an das Beatmungsgerät angepasst sein, so dass für die vorliegende Schlauchgeometrie die Steuereinheit besonders zuverlässig das Inspirations- oder Exspirationsventil regeln kann.

Vorzugsweise ist das mindestens eine Sensorelement innerhalb des Schlauchsystems angeordnet und mit der Steuereinheit des Beatmungsgerätes signaltechnisch verbunden.

Das Inspirations- oder Exspirationsventil ist vorzugsweise in dem Schlauchsystem angeordnet, um unmittelbar am Schlauchsystem einen Fluss des Atemgases aus dem Beatmungskreis des Beatmungsgerätes heraus zu ermöglichen.

In einer vorteilhaften Ausführungsform ist eine Verbindung des Schlauchsystems mit dem Patienten über Prongs bereitgestellt ist. Eine derartige Verbindung über Prongs kann sich während des Tragens dieser Prongs hinsichtlich der dabei bereitgestellt Leckage verändern, so dass das erfindungsgemäße Beatmungsgerät besonders vorteilhaft für die Verwendung derartiger Prongs ist. So kann auch bei einer über die Zeit veränderlichen Leckage der vorbestimmte Maximaldruck vermieden werden.

Gemäß einem dritten Aspekt der Offenbarung wird zur Lösung der oben genannten Aufgabe ein Verfahren zur Regelung eines durch ein Beatmungsgerät bereitgestellten Beatmungsdrucks während einer High-Flow-Sauerstofftherapie über ein Schlauchsystem vorgeschlagen. Das erfindungsgemäße Verfahren weist die folgenden Schritte auf:
- Bereitstellen mindestens eines ansteuerbaren Inspirations- oder Exspirationsventils, insbesondere eines elektrisch ansteuerbaren Inspirations- oder Exspirationsventils, zum Ermöglichen eines Flusses eines Atemgases aus einem Beatmungskreis des Beatmungsgerätes heraus;
- Bestimmen und Ausgeben einer Messgröße innerhalb des Schlauchsystems, wobei die Messgröße einen Gasfluss innerhalb des Schlauchsystems indiziert;
- Regeln eines durch das Beatmungsgerätes bereitgestellten Beatmungsdruckes über das mindestens eine Inspirations- oder Exspirationsventil derart, dass ein vorbestimmter Maximaldruck in einem vorbestimmten Bereich des Schlauchsystems nicht überschritten wird.

Das Verfahren gemäß dem dritten Aspekt der Offenbarung wird von dem Beatmungsgerät gemäß dem ersten Aspekt der Erfindung ausgeführt, so dass es die Vorteile des Beatmungsgerätes umfasst. Insbesondere erlaubt das Verfahren einen konstant anliegenden Gasfluss am Patienten während der High-Flow-Sauerstofftherapie während gleichzeitig die Gefahr eines zu hohen Gasdrucks am Patienten vermieden wird.

Gemäß dem vierten Aspekt der Offenbarung wird zur Lösung der oben genannten Aufgabe ein Computerprogramm mit einem Programmcode zur Durchführung eines Verfahrens gemäß dem dritten Aspekt der Offenbarung vorgeschlagen, wenn der Programmcode auf einem Computer, einem Prozessor oder einer programmierbaren Hardwarekomponente ausgeführt wird. Vorzugsweise werden mehrere Schritte des Verfahrens durch einen gemeinsamen Computer, einen gemeinsamen Prozessor oder eine gemeinsame programmierbare Hardwarekomponente ausgeführt. Vorzugsweise sind die einzelnen Schritte dabei zumindest auf Software-Ebene voneinander durch entsprechende Softwareblöcke getrennt. Besonders bevorzugt werden alle Schritte des Verfahrens auf einem gemeinsamen Computer einem gemeinsamen Prozessor oder einer gemeinsamen programmierbaren Hardwarekomponente ausgeführt.

Die Erfindung soll nun anhand von in den Figuren schematisch dargestellten, vorteilhaften Ausführungsbeispielen näher erläutert werden. Von diesen zeigen im Einzelnen:
- Fig. 1: eine schematische Darstellung eines ersten Ausführungsbeispiels eines Beatmungsgerätes gemäß einem ersten Aspekt der Erfindung;
- Fig. 2: eine schematische Darstellung eines zweiten Ausführungsbeispiels eines Beatmungsgerätes gemäß dem ersten Aspekt der Erfindung;
- Fig. 3: eine schematische Darstellung eines Ausführungsbeispiels eines Beatmungssystems gemäß einem zweiten Aspekt Erfindung;
- Fig. 4: ein Flussdiagramm eines Ausführungsbeispiels eines Verfahrens gemäß einem dritten Aspekt der Offenbarung

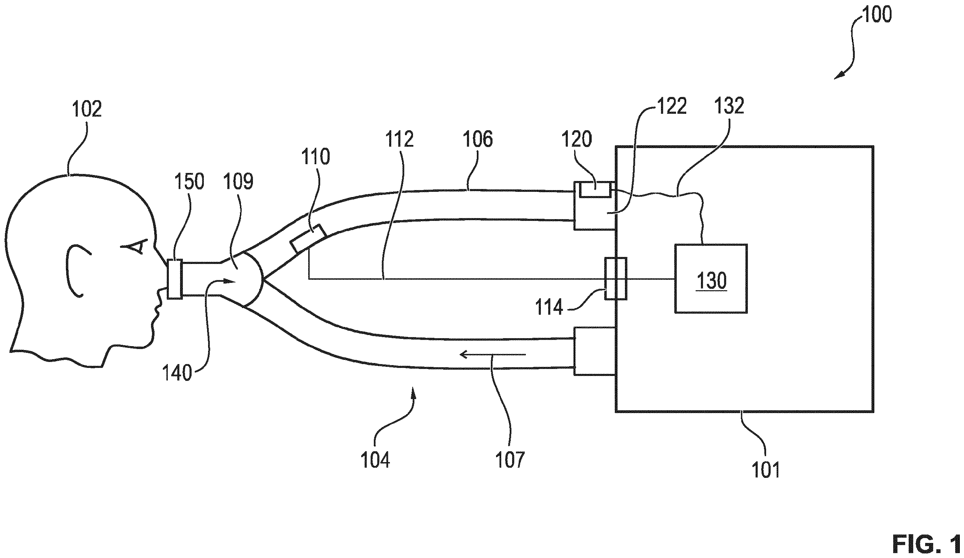

Fig. 1 zeigt eine schematische Darstellung eines ersten Ausführungsbeispiels eines Beatmungsgerätes 100 gemäß einem ersten Aspekt der Erfindung.

Das erfindungsgemäße Beatmungsgerät 100 ist zur Beatmung eines Patienten 102 durch eine High-Flow-Sauerstofftherapie über ein Schlauchsystem 104 ausgebildet.

Das Beatmungsgeräts 100 umfasst mindestens ein Sensorelement 110, mindestens ein ansteuerbares Inspirations- oder Exspirationsventil 120 und eine Steuereinheit 130.

Das mindestens eine Sensorelement 110 ist im dargestellten Ausführungsbeispiel ein Flusssensor, der angeordnet und ausgebildet ist, eine Messgröße 112, nämlich vorliegend einen Gasfluss im Bereich des Sensorelementes 110, innerhalb des Schlauchsystems 104 zu bestimmen und auszugeben. Hierfür ist der Flusssensor in einem Exspirationsschlauch 106 des Schlauchsystems 104 an einem zum Patienten 102 weisenden Ende dieses Exspirationsschlauches 106 angeordnet. Der Flusssensor ist vorliegend kabelbasiert mit der Steuereinheit 130 verbunden. In einem nicht dargestellten Ausführungsbeispiel ist das mindestens eine Sensorelement kabellos mit der Steuereinheit verbunden. In einem weiteren nicht dargestellten Ausführungsbeispiel ist das Sensorelement indirekt über eine weitere Verarbeitungseinheit des Beatmungsgerätes mit der Steuereinheit verbunden.

Die Verbindung zwischen Steuereinheit 130 und dem Sensorelement 110 erfolgt über eine Schnittstelle 114 am Gehäuse 101 des Beatmungsgerätes 100. Über diese Schnittstelle 114 werden die bereitgestellten Daten, die die Messgröße 112 indizieren, über ein durch die Steuereinheit 130 auslesbares Signal bereitgestellt.

Das mindestens eine ansteuerbare Inspirations- oder Exspirationsventil 120 ist angeordnet und ausgebildet, einen Fluss eines Atemgases aus einem Beatmungskreis 107 des Beatmungsgerätes 100 heraus zu ermöglichen. In dem dargestellten Ausführungsbeispiel handelt es sich hierbei um ein Exspirationsventil, das im Bereich einer Aufnahme 122 des Beatmungsgerätes 100 für den Exspirationsschlauch 106 ausgebildet ist. Hierdurch ist keine bauliche Veränderung des Schlauchsystems für die Umsetzung des erfindungsgemäßen Ventils 120 erforderlich. In einem nicht dargestellten alternativen oder ergänzenden Ausführungsbeispiel ist das erfindungsgemäße Ventil ein Inspirationsventil, das in einer Aufnahme des Beatmungsgerätes für einen Inspirationsschlauch ausgebildet ist. Das Exspirationsventil 120 in dem dargestellten Ausführungsbeispiel wird derart von der Steuereinheit 130 angesteuert, dass es geschlossen bleibt, solange der vorbestimmte Maximaldruck nicht in dem vorbestimmten Bereich 140 des Schlauchsystems 104 erreicht ist. Erst nachdem über das Sensorelement 110 ein derartiger vorbestimmter Maximaldruck indiziert ist, wird das Exspirationsventil 120 geöffnet und dadurch ein Gasfluss aus dem Beatmungskreis 107 heraus ermöglicht.

Die Steuereinheit 130 ist mit dem Sensorelement 110 über die Schnittstelle 114 verbunden und mit dem Exspirationsventil 120 direkt über ein entsprechendes Ventilkabel 132 verbunden. In einem nicht dargestellten Ausführungsbeispiel ist die Steuereinheit mit dem Inspirations- oder Exspirationsventil kabellos verbunden. In dem dargestellten Ausführungsbeispiel ermöglicht die Verbindung über das Ventilkabel 132 eine elektrische Steuerung, über eine Ventilstellung des Exspirationsventils 120 elektrisch verändert werden kann. In einem nicht dargestellten Ausführungsbeispiel erfolgt die Ansteuerung des Inspirations- oder Exspirationsventils durch die Steuereinheit über ein pneumatisches Mittel zur Ansteuerung und/oder über ein mechanisches Mittel zur Ansteuerung. Die Steuereinheit 130 ist ausgebildet, einen durch das Beatmungsgerät 100 bereitgestellten Beatmungsdruck innerhalb des Beatmungskreises 107 derart über das mindestens eine Sensorelement 110 und das mindestens eine Inspirations- oder Exspirationsventil 120 zu regeln, dass ein vorbestimmter Maximaldruck in dem vorbestimmten Bereich 140 des Schlauchsystems 104 nicht überschritten wird. Der vorbestimmte Bereich 140 ist hierbei ein Bereich am Y-Stück 109 des Schlauchsystems 104. Dadurch dass das Sensorelement 110 nahe dem Y-Stück 109 angeordnet ist, kann durch die ausgegebene Messgröße 112 auf den Gasdruck im Bereich des Y-Stücks 109 geschlossen werden.

Der vorbestimmte Maximaldruck liegt in dem dargestellten Ausführungsbeispiel zwischen 5mbar und 25 mbar, insbesondere zwischen 10 mbar und 20 mbar, besonders bevorzugt bei etwa 12 mbar.

Das Schlauchsystem 104 ist in dem dargestellten Ausführungsbeispiel über eine Patientenschnittstelle 150 mit dem Patienten 102 verbunden. Die Patientenschnittstelle 150 ist lediglich eine Verbindung mit einem Nasenbereich des Patienten 102. Hierbei handelt es sich um eine Verbindung, die eine Leckage zwischen Schlauchsystem 104 und Patient 102 zulässt. In einem nicht dargestellten Ausführungsbeispiel handelt es sich bei der Patientenschnittstelle um eine Verbindung mit dem Nasenbereich und einem Mundbereich des Patienten. Vorzugsweise handelt es sich bei der Patientenschnittstelle 150 um sogenannte Prongs, die in ein jeweiliges Nasenloch des Patienten eingesetzt werden und damit direkt das zur Beatmung des Patienten 102 zu nutzende Gas in die Atemwege des Patienten 102 einbringen.

Der Beatmungskreis 107 wird vorzugsweise über ein nicht dargestelltes Gebläse das Beatmungsgerätes 100 bereitgestellt. Die Leistung dieses Gebläses oder einer ähnlichen einen Gasfluss bereitstellen Vorrichtung wird in dem dargestellten Ausführungsbeispiel nicht durch die Steuereinheit 130 beim Vorliegen des vorbestimmten Maximaldruck in dem vorbestimmten Bereich 140 verändert. Das Regeln durch die Steuereinheit 130 basiert vorliegend ausschließlich auf der Steuerung des Inspirations- oder Exspirationsventils 120.

Die Steuereinheit 130 ist innerhalb des Gehäuses 101 des Beatmungsgerätes 100 angeordnet. Das Inspirations- oder Exspirationsventil 120 ist innerhalb der Aufnahme 122 und damit ebenfalls am Gehäuse 101 angeordnet. Das Sensorelement 110 ist an dem Schlauchsystem 104 und damit außerhalb des Gehäuses 101 angeordnet. In einem nicht dargestellten Ausführungsbeispiel ist das Sensorelement ebenfalls innerhalb und/oder an dem Gehäuse des erfindungsgemäßen Beatmungsgerätes angeordnet. Eine größere Distanz zwischen dem Sensorelement und dem vorbestimmten Bereich des Schlauchsystems als in dem dargestellten Ausführungsbeispiel führt dazu, dass die bestimmte Messgröße über eine entsprechende Umrechnung in eine voraussichtlich im vorbestimmten Bereich vorliegende Messgröße umgerechnet werden muss.

Das Inspirations- oder Exspirationsventil 120 verfügt in dem dargestellten Ausführungsbeispiel über eine elektrisch ansteuerbare Klappe, die entweder in einem offenen oder einem geschlossenen Zustand vorliegt, abhängig von der Ansteuerung durch die Steuereinheit 130. Entsprechend kann das vorliegende Exspirationsventil über die elektrische Ansteuerung durch die Steuereinheit 130 zwischen der entsprechenden offenen Ventilstellung und der entsprechenden geschlossenen Ventilstellung umgeschaltet werden. In einem alternativen oder ergänzenden Ausführungsbeispiel ist das entsprechende Ventil derart ausgestaltet, dass es eine Mehrzahl von unterschiedlichen Strömungswiderständen im Rahmen einer Mehrzahl von Ventilstellungen bereitstellen kann. Die genaue Ausgestaltung eines derartigen Ventils ist dem Fachmann bekannt und wird daher im Folgenden nicht detailliert erläutert.

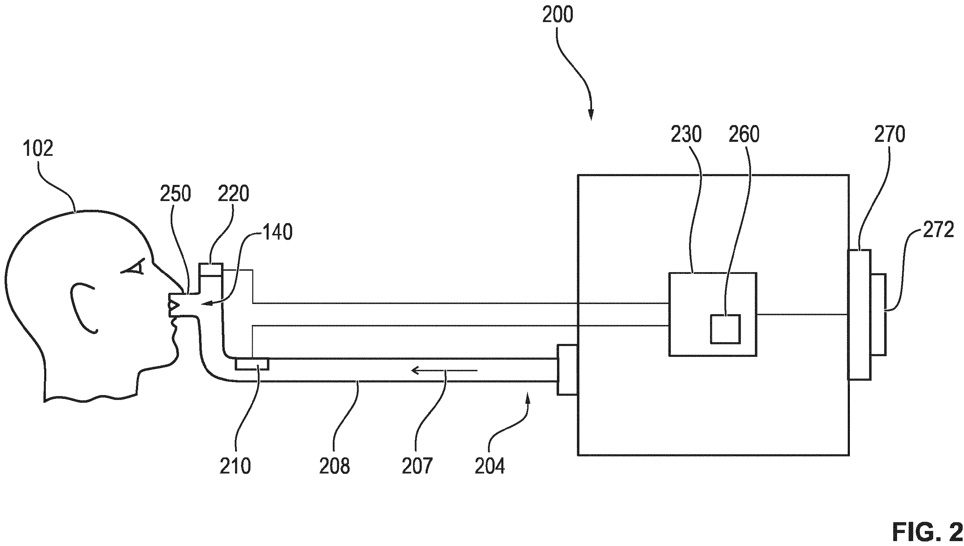

Fig. 2 zeigt eine schematische Darstellung eines zweiten Ausführungsbeispiels eines Beatmungsgerätes 200 gemäß dem ersten Aspekt der Erfindung.

Das Beatmungsgerät 200 unterscheidet sich von dem in Fig. 1 dargestellten Beatmungsgerät 100 dadurch, dass das Schlauchsystem 204 lediglich aus einem Inspirationsschlauch 208 und der endsprechenden Patientenschnittstelle 250 mit dem Patienten 102 besteht. Der komplette bereitgestellte Atemgasfluss 207 soll daher über die Patientenschnittstelle 250, die vorliegend durch Prongs eine Verbindung mit der Nase des Patienten 102 bereitstellt, zum Patienten 102 geführt werden. Der Atemgasfluss 207 bildet in diesem Ausführungsbeispiel einen Beatmungskreis für die dargestellte Struktur des Schlauchsystems 204. Falls eine Leckage zwischen Patientenschnittstelle 205 und Patient 102 zu klein ist und daher der Gasdruck auf den Patienten ein unbedenkliches Maß überschreitet, detektiert das mindestens eine Sensorelement 210, bei dem es sich vorliegend um einen Drucksensor handelt, einen solchen Anstieg des Gasdrucks. Falls hierbei der vorbestimmte Maximaldruck in dem vorbestimmten Bereich 140, nämlich vorliegend in dem Bereich der Patientenschnittstelle 250 überschritten ist oder voraussichtlich überschritten wird, öffnet das Inspirationsventil 220, welches an einer Verlängerung des Inspirationsschlauch 208 ausgebildet ist. Das Inspirationsventil 220 weist, wie bereits das Exspirationsventil aus Fig. 1, eine Klappe auf, die abhängig von einer elektrischen Ansteuerung nur zwischen einer offenen Stellung und einer geschlossenen Stellung wechseln kann.

Im Normalbetrieb des Beatmungsgerätes 200 ist der bereitgestellte Atemgasfluss 207 so gewählt, dass an der Patientenschnittstelle 250 das komplette bereitgestellte Atemgas austritt. Dabei wird ein Teil des Atemgases in die Nase des Patienten 102 geführt und der restliche Teil wird über eine Leckage zwischen Patientenschnittstelle 250 und Patient 102 an diesem Patienten vorbeigeführt. Nur wenn die Prongs der Patientenschnittstelle 250 zu eng an der Nase anliegen und die Leckage entsprechend zu gering ist, steigt der Druck innerhalb des Schlauchsystems 204 an und kann ohne eine entsprechende Regelung der Steuereinheit 230 zu einem für die Gesundheit des Patienten 102 bedenklichen Druck oberhalb des vorbestimmten Maximaldrucks führen.

In der Steuereinheit 230 ist eine Kalibrierungsinformation 260 dauerhaft hinterlegt. Die Steuereinheit regelt das entsprechende Ventil 220 abhängig von der Kalibrierungsinformation 260. Die Kalibrierungsinformation stellt dabei eine Zuordnung zwischen dem durch den Drucksensor 210 gemessenen Druck und dem in dem vorbestimmten Bereich 140 vorliegenden Druck dar. Die Kalibrierungsinformation 260 ist dabei in einem nicht dargestellten Speichermodul der Steuereinheit 230 hinterlegt.

Weiterhin umfasst das Beatmungsgerät 200 eine Ausgabeeinheit 270, die über ein Display 272 anzeigt, ob eine Ansteuerung des Inspirations- oder Exspirationsventils 220 zum Vermeiden des Maximaldruck in dem vorbestimmten Bereich 140 erfolgt ist. Hierdurch kann ein Nutzer des Beatmungsgeräts 200, wie etwa medizinisches Fachpersonal, erkennen, dass möglicherweise aktuell das Schlauchsystem 204 nicht wie gewünscht locker über die Patientenschnittstelle 250 an dem Patienten 102 anliegt. In diesem Sinne kann über die Ausgabeeinheit 270 direkt eine den Druck innerhalb des Schlauchsystems 204 regulierende Handlung des medizinischen Fachpersonals ausgelöst werden.

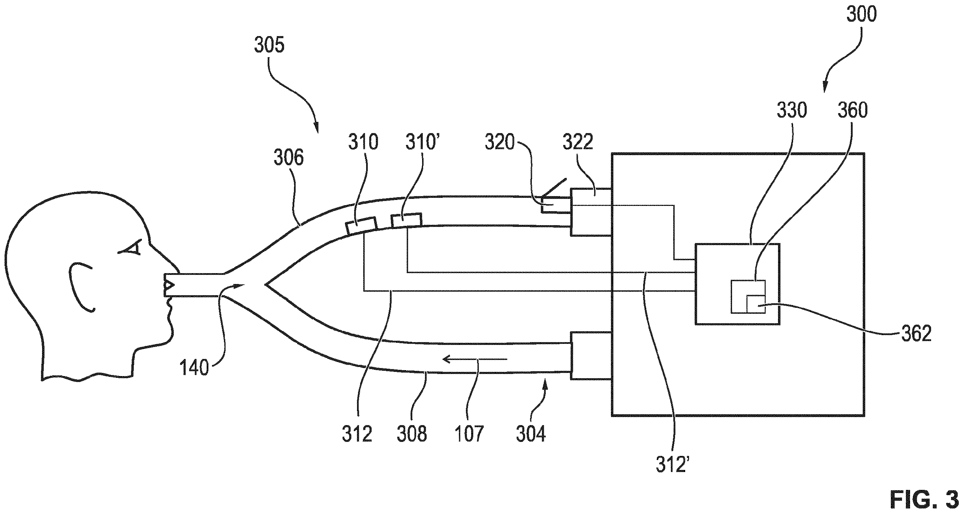

Fig. 3 zeigt eine schematische Darstellung eines Ausführungsbeispiels eines Beatmungssystems 305 gemäß einem zweiten Aspekt Erfindung.

Das Beatmungssystem 305 umfasst ein Beatmungsgerät 300 gemäß dem ersten Aspekt der Erfindung und das Schlauchsystem 304. Das Schlauchsystem 304 ist dabei zum Bereitstellen des Beatmungskreises 107 an das Beatmungsgerät 300 angeschlossen. Dabei weist das Schlauchsystem 304 die gleiche Struktur auf wie das Schlauchsystem 104 aus Fig. 1 und weist demnach einen Exspirationsschlauch 306 und einen Inspirationsschlauch 308 auf.

Im Unterschied zu dem Beatmungsgerät 100 aus Fig. 1 weist das Beatmungsgerät 300 zwei Sensorelemente 310, 310` auf, die beide am Exspirationsschlauch 306 angeordnet sind. Es handelt sich hierbei um einen Flusssensor und einen Drucksensor. Durch die Kombination der beiden bestimmten Messgrößen 312, 312` kann besonders zuverlässig durch die Steuereinheit 330 festgestellt werden, ob das Inspirations- oder Exspirationsventil 320 angesteuert werden muss, um den vorbestimmten Maximaldruck in dem vorbestimmten Bereich 140 zu vermeiden.

Hierfür greift die Steuereinheit 330 auf einer hinterlegte Kalibrierungsinformation 360 zu, die in dem dargestellten Ausführungsbeispiel einen Schlauchwiderstand 362, insbesondere einen vorbestimmten Schlauchwiderstand, des Schlauchsystems 304 umfasst. Vorzugsweise wird durch das Beatmungsgerätes automatisiert erkannt, welches Schlauchsystem 304 an das Beatmungsgeräts geschaltet wird und abhängig von dem hinterlegten vorbestimmten Schlauchwiderstand des erkannten Schlauchsystems 304 wird die entsprechende dem Schlauchwiderstand zugeordnete Kalibrierungsinformation verwendet, die für den vorliegenden Schlauchwiderstand 362 eine zuverlässige Zuordnung von gemessenem Gasfluss und von gemessenem Gasdruck zu dem in dem vorbestimmten Bereich 140 vorliegenden Gasdruck bereitstellt. Die automatisierte Erkennung des vorliegenden Schlauchsystems 304 erfolgt beispielsweise über das Auslesen einer Identifikationsnummer im Bereich einer Aufnahme 322 eines jeweiligen Schlauchs durch das Beatmungsgerät 300. Die Verwendung von zwei Sensoren für das erfindungsgemäße Beatmungsgerät kann eine Ausfallsicherheit gegen den Ausfall eines Sensors erhöhen. So können die beiden bestimmten Messgrößen beispielsweise miteinander verglichen werden, um einen Fehler bei der Bestimmung einer Messgröße zu detektieren und ein entsprechendes Fehlersignal auszugeben.

Vorliegend wird als Ventil ein Exspirationsventil 320 verwendet, dass am zum Beatmungsgeräts 300 weisenden Ende des Exspirationsschlauchs 306 angeordnet ist. Das Ventil ist in Fig. 3 in einem geöffneten Zustand, also mit geöffneter Klappe dargestellt. Folglich wurde in dem dargestellten Zustand der vorbestimmte Maximaldruck in dem vorbestimmten Bereich 140 erreicht oder es wurde festgestellt, dass dieser Maximaldruck wahrscheinlich bei unverändertem Betrieb erreicht wird, so dass über das Exspirationsventil 320 ein Gasfluss aus dem Schlauchsystem 304 in einer Umgebung des Beatmungsgerätes 300 abgelassen wird.

Erfindungsgemäß sind für die Position des jeweiligen mindestens einen Sensorelementes und des mindestens einen ansteuerbaren Inspirations- oder Exspirationsventils sämtliche Positionen an dem jeweils genutzten Schlauchsystem oder innerhalb einer Gasführung in dem Gehäuse des erfindungsgemäßen Beatmungsgerätes möglich. So muss anhand der Messgröße lediglich auf den in dem vorbestimmten Bereich vorliegenden Druck geschlossen werden können, was bei entsprechender Kalibrierung auch bei einem gewissen räumlichen Abstand zu dem vorbestimmten Bereich möglich ist.

Das entsprechende Ventil muss lediglich eine pneumatische Verbindung zu dem vorbestimmten Bereich des Schlauchsystems aufweisen, um dort eine Regulierung des vorliegenden Drucks zu ermöglichen. Dies ist auch beispielsweise für ein Ventil innerhalb des Gehäuses des Beatmungsgerätes möglich.

Die Kommunikation zwischen den Sensorelementen 310, 310` und der Steuereinheit 330, sowie zwischen dem Exspirationsventil 320 und der Steuereinheit 330 erfolgt in dem dargestellten Ausführungsbeispiel kabelbasiert. In einem nicht dargestellten Ausführungsbeispiel erfolgt die Kommunikation zumindest teilweise kabellos.

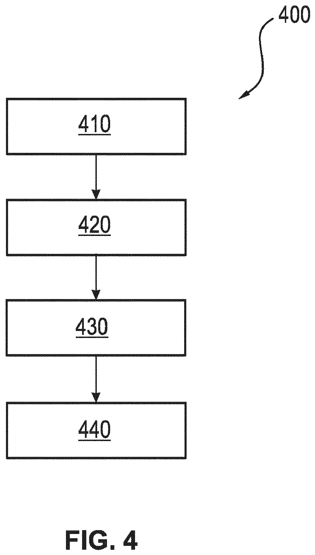

Fig. 4 zeigt ein Flussdiagramm eines Ausführungsbeispiels eines Verfahrens 400 gemäß einem dritten Aspekt der Offenbarung.

Das offenbarungsgemäße Verfahren 400 ist zur Regelung eines durch ein Beatmungsgerät bereitgestellten Beatmungsdrucks während einer High-Flow-Sauerstofftherapie über ein Schlauchsystem ausgebildet. Hierfür weist es die im Folgenden dargestellten Schritte auf.

Ein erster Schritt 410 umfasst ein Bereitstellen mindestens eines ansteuerbaren Inspirations- oder Exspirationsventils, insbesondere eines elektrisch ansteuerbaren Inspirations- oder Exspirationsventils, zum Ermöglichen eines Flusses eines Atemgases aus einem Beatmungskreis des Beatmungsgerätes heraus.

Ein darauffolgender Schritt 420 umfasst ein Bestimmen einer Messgröße innerhalb des Schlauchsystems, wobei die Messgröße einen Gasfluss innerhalb des Schlauchsystems indiziert.

Ein unmittelbar auf den Schritt 420 folgender Schritt 430 umfasst ein Ausgeben dieser Messgröße.

Ein abschließender Schritt 440 umfasst ein Regeln eines durch das Beatmungsgerät bereitgestellten Beatmungsdruckes über das mindestens eine Inspirations- oder Exspirationsventil derart, dass ein vorbestimmter Maximaldruck in einem vorbestimmten Bereich des Schlauchsystems nicht überschritten wird.

Der erste Schritt 410 wird typischerweise bei der Herstellung des Beatmungsgerätes und/oder eines Systems aus Beatmungsgerät und Schlauchsystem ausgeführt. Nach der Herstellung dieses Beatmungsgerätes oder dieses Systems folgen nur noch die Schritte 420, 430 und 440.

Dabei werden die Schritte 420 und 430, also das Bestimmen und Ausgeben der Messgröße vorzugsweise unmittelbar hintereinander ausgeführt. Der zeitliche Versatz zwischen diesen beiden Schritten beträgt vorzugsweise weniger als 20 Sekunden, insbesondere weniger als 10 Sekunden, vorzugsweise weniger als 5 Sekunden.

Die beiden Schritte 420 und 430 werden vorzugsweise unabhängig von dem Schritt 440 in wiederkehrenden zeitlichen Abständen ausgeführt. Vorzugsweise wird die Messgröße nach weniger als 1 Minute, insbesondere nach weniger als 30 Sekunden, besonders bevorzugt nach weniger als 5 Sekunden erneut bestimmt und ausgegeben.

Der abschließender Schritt 440 wird vorzugsweise nur ausgeführt, wenn der ausgegebene Messwert anzeigt, dass der vorbestimmte Maximaldruck überschritten ist oder voraussichtlich überschritten wird. Falls der vorbestimmte Maximaldruck angesichts der bestimmten Messgröße voraussichtlich nicht erreicht wird, findet vorzugsweise keine Regelung entsprechend dem Schritt 440 statt.

### Bezugszeichenliste

- 100, 200, 300: Beatmungsgerät
- 101: Gehäuse
- 102: Patient
- 104, 204, 304: Schlauchsystem
- 106, 306: Exspirationsschlauch
- 107: Beatmungskreis
- 109: Y-Stück
- 110, 210, 310, 310`: Sensorelement
- 112, 312, 312`: Messgröße
- 114: Schnittstelle für das Sensorelement
- 120, 220, 320: Inspirations- oder Exspirationsventil
- 122, 322: Aufnahme
- 130, 230, 330: Steuereinheit
- 132: Ventilkabel
- 140: vorbestimmter Bereich
- 150, 250: Patientenschnittstelle
- 207: Atemgasfluss
- 208, 308: Inspirationsschlauch
- 260, 360: Kalibrierungsinformation
- 270: Ausgabeeinheit
- 272: Display
- 305: Beatmungssystem
- 362: vorbestimmter Schlauchwiderstand
- 400: Verfahren
- 410, 420, 430, 440: Verfahrensschritte

## Patentansprüche

1. Beatmungsgerät (100) zur Beatmung eines Patienten (102) durch eine High-Flow-Sauerstofftherapie über ein Schlauchsystem (104),
wobei das Beatmungsgerät (100) aufweist
- mindestens ein Sensorelement (110), das angeordnet und ausgebildet ist, eine Messgröße (112) innerhalb des Schlauchsystems (104) zu bestimmen und auszugeben, wobei die Messgröße (112) einen Gasfluss innerhalb des Schlauchsystems (104) indiziert,
- mindestens ein ansteuerbares Inspirations- oder Exspirationsventil (120), das angeordnet und ausgebildet ist, einen Fluss eines Atemgases aus einem Beatmungskreis (107) des Beatmungsgerätes (100) heraus zu ermöglichen, und
- eine Steuereinheit (130),
**dadurch gekennzeichnet, dass**
die Steuereinheit (130) eingerichtet ist, einen durch das Beatmungsgerät (100) bereitgestellten Beatmungsdruck derart über das mindestens eine Sensorelement (110) und das mindestens eine Inspirations- oder Exspirationsventil (120) zu regeln, dass ein vorbestimmter Maximaldruck in einem vorbestimmten Bereich (140) des Schlauchsystems (104) nicht überschritten wird, wobei die Steuereinheit (130) eingerichtet ist, die Regelung des bereitgestellten Beatmungsdruckes auszuführen, ohne hierfür eine Leistung eines Gebläses des Beatmungsgerätes (100) oder dergleichen zu verändern.

2. Beatmungsgerät (100) gemäß Anspruch 1,
wobei das Beatmungsgerät (100) dazu ausgebildet ist, das mindestens eine Inspirations- oder Exspirationsventil (120) geschlossen zu halten, falls der vorbestimmte Maximaldruck nicht in dem vorbestimmten Bereich (140) des Schlauchsystems (104) erreicht ist.

3. Beatmungsgerät (100) gemäß Anspruch 1 oder 2,
wobei das mindestens eine Sensorelement (110) innerhalb eines Exspirationsschlauchs (106) angeordnet ist.

4. Beatmungsgerät (100) gemäß mindestens einem der vorhergehenden Ansprüche,
wobei das Inspirations- oder Exspirationsventil (120) ein elektrisch ansteuerbares Inspirations- oder Exspirationsventil ist.

5. Beatmungsgerät (100) gemäß mindestens einem der vorhergehenden Ansprüche,
wobei das mindestens eine Sensorelement (110) ein Drucksensor und/oder ein Flusssensor ist.

6. Beatmungsgerät (200) gemäß mindestens einem der vorhergehenden Ansprüche,
wobei die Steuereinheit (230) weiterhin ausgebildet ist, den bereitgestellten Beatmungsdruck basierend auf einer vorbestimmten Kalibrierungsinformation (260) zu regeln.

7. Beatmungsgerät (200) gemäß Anspruch 5 und 6,
wobei die Kalibrierungsinformation (260) einen Zusammenhang zwischen dem in dem vorbestimmten Bereich des Schlauchsystems (204) vorliegenden Druck und dem durch den Flusssensor als das mindestens eine Sensorelement (110) gemessenen Gasfluss indiziert.

8. Beatmungsgerät (300) gemäß Anspruch 6 oder 7,
wobei die Kalibrierungsinformation (360) weiterhin auf einem Schlauchwiderstand (362), insbesondere einem vorbestimmten Schlauchwiderstand, des Schlauchsystems (304) basiert.

9. Beatmungsgerät (100) gemäß mindestens einem der vorhergehenden Ansprüche,
wobei der vorbestimmte Bereich (140) des Schlauchsystems (104) ein zum Patienten (102) weisendes Ende eines Inspirations- oder Exspirationsschlauchs (106) ist.

10. Beatmungsgerät (100) gemäß mindestens einem der vorhergehenden Ansprüche,
wobei das mindestens eine Inspirations- oder Exspirationsventil (120) zusammen mit der Steuereinheit (130) in einem Gehäuse (101) des Beatmungsgerätes (100) angeordnet ist.

11. Beatmungsgerät (100) gemäß mindestens einem der vorhergehenden Ansprüche,
wobei das mindestens eine Inspirations- oder Exspirationsventil (120) nur in einer offenen Ventilstellung und in einer geschlossenen Ventilstellung vorliegen kann.

12. Beatmungssystem (305) zur Beatmung eines Patienten (102) durch eine High-Flow-Sauerstofftherapie, mit
- einem Beatmungsgerät (300) gemäß mindestens einem der vorhergehenden Ansprüche, und
- einem Schlauchsystem (304) mit einem Inspirationsschlauch (308) und einem Exspirationsschlauch (306), wobei das Schlauchsystem (304) zum Bereitstellen eines Beatmungskreises (107) an dem Beatmungsgerät (300) angeschlossen ist.

13. Beatmungssystem (305) gemäß Anspruch 12,
wobei eine Verbindung des Schlauchsystems (304) mit dem Patienten (102) über Prongs bereitgestellt ist.

## Claims

1. Ventilator (100) for ventilating a patient (102) by high-flow oxygen therapy via a hose system (104),
wherein the ventilator (100) has
- at least one sensor element (110) which is arranged and designed to determine and output a measured variable (112) within the hose system (104), wherein the measured variable (112) indicates a gas flow within the hose system (104),
- at least one controllable inspiration or expiration valve (120) which is arranged and designed to allow a flow of a respiratory gas out of a ventilation circuit (107) of the ventilator (100), and
- a control unit (130),
**characterized in that**
the control unit (130) is configured to regulate a ventilation pressure, provided by the ventilator (100), via the at least one sensor element (110) and the at least one inspiration or expiration valve (120), such that a predetermined maximum pressure in a predetermined region (140) of the hose system (104) is not exceeded, wherein the control unit (130) is configured to carry out the regulation of the provided ventilation pressure without changing a power of a blower of the ventilator (100) or the like.

2. Ventilator (100) according to claim 1,
wherein the ventilator (100) is designed to keep the at least one inspiration or expiration valve (120) closed if the predetermined maximum pressure is not reached in the predetermined region (140) of the hose system (104).

3. Ventilator (100) according to claim 1 or 2,
wherein the at least one sensor element (110) is arranged within an expiration hose (106).

4. Ventilator (100) according to at least one of the preceding claims,
wherein the inspiration or expiration valve (120) is an electrically controllable inspiration or expiration valve.

5. Ventilator (100) according to at least one of the preceding claims,
wherein the at least one sensor element (110) is a pressure sensor and/or a flow sensor.

6. Ventilator (200) according to at least one of the preceding claims,
wherein the control unit (230) is furthermore designed to regulate the provided ventilation pressure on the basis of predetermined calibration information (260).

7. Ventilator (200) according to claims 5 and 6,
wherein the calibration information (260) indicates a relationship between the pressure, present in the predetermined region of the hose system (204), and the gas flow measured by the flow sensor as the at least one sensor element (110).

8. Ventilator (300) according to claim 6 or 7,
wherein the calibration information (360) is further based upon a hose resistance (362) - in particular, a predetermined hose resistance - of the hose system (304).

9. Ventilator (100) according to at least one of the preceding claims,
wherein the predetermined region (140) of the hose system (104) is an end, pointing towards the patient (102), of an inspiration or expiration hose (106).

10. Ventilator (100) according to at least one of the preceding claims,
wherein the at least one inspiration or expiration valve (120) is arranged together with the control unit (130) in a housing (101) of the ventilator (100).

11. Ventilator (100) according to at least one of the preceding claims,
wherein the at least one inspiration or expiration valve (120) can be only in an open valve position and in a closed valve position.

12. Ventilation system (305) for ventilating a patient (102) by high-flow oxygen therapy, having
- a ventilator (300) according to at least one of the preceding claims, and
- a hose system (304) having an inspiration hose (308) and an expiration hose (306), wherein the hose system (304) is connected to the ventilator (300) for providing a ventilation circuit (107).

13. Ventilation system (305) according to claim 12,
wherein a connection of the hose system (304) to the patient (102) is provided via prongs.

## Revendications

1. Respirateur (100) permettant de faire respirer un patient (102) par une oxygénothérapie à haut débit par l'intermédiaire d'un système de tubes (104),
dans lequel le respirateur (100) présente
- au moins un élément formant capteur (110) qui est disposé et configuré pour déterminer et émettre une grandeur de mesure (112) à l'intérieur du système de tubes (104), dans lequel la grandeur de mesure (112) indique un écoulement de gaz à l'intérieur du système de tubes (104),
- au moins une soupape d'inspiration ou d'expiration (120) pouvant être commandée qui est disposée et conçue pour permettre un écoulement d'un gaz respiratoire hors d'un circuit respiratoire (107) du respirateur (100), et
- une unité de commande (130),
**caractérisé en ce que**
l'unité de commande (130) est configurée pour réguler une pression de respiration fournie par le respirateur (100) par l'intermédiaire de l'au moins un élément formant capteur (110) et de l'au moins une soupape d'inspiration ou d'expiration (120) de telle sorte qu'une pression maximale prédéterminée n'est pas dépassée dans une zone prédéterminée (140) du système de tubes (104), dans lequel l'unité de commande (130) est configurée pour exécuter la régulation de la pression de respiration fournie sans modifier pour cela une puissance d'une soufflante du respirateur (100) ou similaire.

2. Respirateur (100) selon la revendication 1,
dans lequel le respirateur (100) est conçu pour maintenir l'au moins une soupape d'inspiration ou d'expiration (120) fermée si la pression maximale prédéterminée n'est pas atteinte dans la zone prédéterminée (140) du système de tubes (104).

3. Respirateur (100) selon la revendication 1 ou 2,
dans lequel l'au moins un élément formant capteur (110) est disposé à l'intérieur d'un tube d'expiration (106).

4. Respirateur (100) selon au moins l'une des revendications précédentes,
dans lequel la soupape d'inspiration ou d'expiration (120) est une soupape d'inspiration ou d'expiration pouvant être commandée électriquement.

5. Respirateur (100) selon au moins l'une des revendications précédentes,
dans lequel l'au moins un élément formant capteur (110) est un capteur de pression et/ou un capteur d'écoulement.

6. Respirateur (200) selon au moins l'une des revendications précédentes,
dans lequel l'unité de commande (230) est en outre configurée pour réguler la pression de respiration fournie sur la base d'une information d'étalonnage (260) prédéterminée.

7. Respirateur (200) selon les revendications 5 et 6,
dans lequel l'information d'étalonnage (260) indique une relation entre la pression présente dans la zone prédéterminée du système de tubes (204) et l'écoulement de gaz mesuré par le capteur d'écoulement en tant que l'au moins un élément formant capteur (110).

8. Respirateur (300) selon la revendication 6 ou 7,
dans lequel l'information d'étalonnage (360) est en outre basée sur une résistance de tube (362), en particulier une résistance de tube prédéterminée, du système de tubes (304).

9. Respirateur (100) selon au moins l'une des revendications précédentes,
dans lequel la zone prédéterminée (140) du système de tubes (104) est une extrémité d'un tube d'inspiration ou d'expiration (106) orientée vers le patient (102).

10. Respirateur (100) selon au moins l'une des revendications précédentes,
dans lequel l'au moins une soupape d'inspiration ou d'expiration (120) est disposée conjointement avec l'unité de commande (130) dans un boîtier (101) du respirateur (100).

11. Respirateur (100) selon au moins l'une des revendications précédentes,
dans lequel l'au moins une soupape d'inspiration ou d'expiration (120) peut être présente uniquement dans une position de soupape ouverte et dans une position de soupape fermée.

12. Système de respiration (305) permettant de faire respirer un patient (102) par une oxygénothérapie à haut débit, comportant
- un respirateur (300) selon au moins l'une des revendications précédentes, et
- un système de tubes (304) comportant un tube d'inspiration (308) et un tube d'expiration (306), dans lequel le système de tubes (304) est raccordé au respirateur (300) pour fournir un circuit respiratoire (107).

13. Système de respiration (305) selon la revendication 12,
dans lequel une liaison du système de tubes (304) au patient (102) est fournie par l'intermédiaire de crochets.
